# EUROPÄISCHE PATENTSCHRIFT

(11) **EP 2 023 986 B1**
(45) Veröffentlichungstag und Bekanntmachung des Hinweises auf die Patenterteilung: **22.02.2012**
(21) Anmeldenummer: 07722513.4
(22) Anmeldetag: 06.06.2007
(51) Int. Cl.: A61M 11/00, A61M 15/00

(54) **DOSIERINHALATOR**
DOSAGE INHALER
INHALATEUR-DOSEUR

(30) Priorität: 07.06.2006 DE 102006026786
(43) Veröffentlichungstag der Anmeldung: 18.02.2009
(73) Patentinhaber: Kern, Joachim, 63820 Elsenfeld (DE)
(72) Erfinder: Kern, Joachim, 63820 Elsenfeld (DE)
(74) Vertreter: Pöhner, Wilfried Anton
(86) Internationale Anmeldenummer: PCT/DE2007/001001
(87) Internationale Veröffentlichungsnummer: WO 2007/140761

(56) Entgegenhaltungen:
- WO-A-01/85241
- DE-A1- 19 934 582
- FR-A1- 2 783 431
- US-A- 4 001 650
- US-A- 5 865 171
- US-A1- 2003 192 532

## Beschreibung

Die Erfindung bezieht sich auf einen Dosierinhalator, bestehend aus einem Wirkflüssigkeitsbehälter, in dem sich eine Flüssigkeit mit einem darin gelösten Wirkstoff befindet, sowie einem Vernebler, durch den Flüssigkeit in ein Aerosol umwandelbar und in einen Aerosoldom einbringbar ist, in welchem eine Prallplatte angeordnet ist und an welchem ein Zuluftrohr sowie ein Abluftrohr angeschlossen ist sowie einer elektronischen Steuerung, die den Vernebler und die Benutzerbefehlsausgabe ansteuert, wobei der Vernebler intermittierend betreibbar ist und eine Benutzerbefehlsausgabe für den Benutzer bemerkbar ist und die Erzeugung der innerhalb und unterhalb des Aerosoldomes befindlichen Aerosolmenge von deren Inhalation zeitlich trennbar ist.

Das inhalieren von Wirkstoffen, die in Wasser gelöst und als feiner Nebel eingeatmet werden, ist eine seit langem bekannte Methode zur Verabreichung von Wirkstoffen, insbesondere im medizinischen Bereich. Je feiner die Tröpfchen sind, desto größer ist der Enteil, der mit der Atemluft bis tief in die Lunge- hinein befördert wird. Aktueller Stand der Technik ist die Erzeugung eines Nebels durch eine Ultraschallschwingeinheit. Sie wird entweder am Boden des Gefäßes mit der Wirkflüssigkeit angeordnet und erzeugt auf der Oberfläche dieser Flüssigkeit einen Sprudel, der so feine Nebel absondert, dass diese sich wie Rauch verhalten und deshalb sehr leicht mit dem Luftstrom mitgezogen werden können. Dabei ist es erforderlich, dass zur Separierung von Aerosoltröpfchen, größer etwa 10 Mikrometer der Luftstrom wenigstens zweimal umgelenkt wird. Dazu dient eine Prallplatte, die oberhalb des Ultraschallsprudels angeordnet ist. Als am wir kungsvollsten hat sich ein nach unten offener, hohler Kegelstumpf erwiesen. In diesen kegelstumpf hinein steigen die Tröpfchen auf und werden in der Mitte des Kegels in Wirbel versetzt, wobei sich die größeren Tropfen aus dem Nebel lösen und in den Wirkflüssigkeitsbehälter zurückfallen. Nur die allerfeinsten Tropfen werden mit dem Luftstrom mitgerissen und um die Kante der Prallplatte herum entweder durch einen schmalen Schlitz oder durch kalibrierte Öffnungen hindurch in den Abluftkanal befördert. An dieser Stelle werden weitere, unerwünscht große Tröpfchen ausgeschieden.

Dabei hat sich das Prinzip der Wirkstoffbeförderung in die Lunge mittels eines Aerosols durch die mit Ultraschallverneblern möglichen, sehr kleinen Tröpfchen als besonders effizient herausgestellt. Es kann nachgewiesen werden, dass Tröpfchen unterhalb einer Mindestgröße vollständig in den Lungenblasen absorbiert werden und in das Blut transferiert werden.

Ein gravierender Schwachpunkt ist jedoch die exakte Dosierung der Wirkstoffmenge. Wenn es auch ein prinzipieller Vorteil des Inhalierens ist, dass im Vergleich zu allen anderen Methoden der Verabreichung von Arzneimitteln absolut gesehen sehr geringe Menge benötigt werden, ist es eine vorwiegend nur psychologische Problematik, dass von der Wirkflüssigkeit stets ein Rest in der Größenordnung von einstelligen Prozenten im Wirkstoffbehälter zurück bleibt. Wenn diese Mindestmenge unterschritten wird, erzeugt die Ultraschallschwingung keinen Sprudel und bildet also auch keine Wassertröpfchen mehr.

Diese Mindestmenge ist jedoch mit guter Genauigkeit im voraus berechenbar und muss deshalb lediglich der zu verabreichenden Dosismenge hinzugeschlagen werden.

Bei der exakten Dosierung der tatsächlich vom Patienten aufgenommenen Wirkstoffmenge ist nach bisherigem Stand der Technik das gravierendste Problem das Atemverhalten des Patienten, das mit den bisher bekannten Geräten weder überwacht noch gesteuert werden kann. Im Unterschied zur oralen Verabreichung - bei der auch ein unwilliger und/oder geschwächter und/oder unkonzentrierter Patient die Arzneimittetdosis entweder komplett aufnimmt oder garnicht absorbiert, jedoch nur in einer praktisch vernachlässigbaren Anzahl von Fällen unbeabsichtigter Weise eine Teilmenge aufnimmt - ist es bei den bisher bekannten Inhalatoren sehr häufig, dass nur ein Teil der in ein Aerosol umgewandelten Wirkstoffmenge tatsächlich vom Patienten aufgenommen wird. Der übrige Teil wird entweder wieder vom Aerosolzustand in eine Flüssigkeit zurückverwandelt, oder lagert sich wegen unzureichender Luftgeschwindigkeit in den Rohrsystemen des Inhalators ab oder gelangt nur bis in den Mund/Rachenraum des Patienten.

Laut WO-01/85241 sollte für die Deposition des Medikamentes in den gewünschten Abschnitten der Atemwege der Patient entsprechend einem vorgegebenen Atemmuster atmen, damit die Strömungsgeschwindigkeiten für die Deposition des Medikamentes nicht ungünstig sind. Das Einhalten der Vorgaben bereitet dem Patienten oft Schwierigkeiten, so dass sich eine nicht unerheblich Menge des Medikamentes in den oberen Atemwegen niederschlägt und somit licht zur Dosis des tatsächlich verabreichten Medikamentes beiträgt.

Bei den einfachen Geräten ohne Zuluft- und Atemventil kann es auch vorkommen, dass der Patient das Aerosol nicht einatmet, sondern aus dem Gerät hinaus in die Umgebung bläst.

Die 199 34 582 A1, Buff, beschreibt einen Inhalator, der über eine Sensorik die Konzentration des Aerosols erfasst und aus der Änderung dieser Konzentration auf den Beginn und das Ende der Einatemphase schließt und in Abhängigkeit davon die Aerosolproduktion steuert. Dadurch kann die Aerosolproduktion mit einer gewissen Trägheit an das tatsächliche Atemverhalten angeglichen werden. Nachteilig ist, dass der Benutzer mittels dreier LED's nur über Gerätezustände informiert wird, jedoch keine eindeutigen Angaben darüber erhält, wann und wie er atmen soll.

Ein weiterer Nachteil ist, dass bei unregelmäßigem oder stoßweisem Atmen Aerosol aus dem Gerät heraus gelangen kann, wodurch die korrekte Dosierung verfälscht wird und unbeteiligte Dritte unerwünschter Weise dem Wirkstoff ausgesetzt werden können.

Auf dieser Basis hat es sich die Erfindung zur Aufgabe gemacht, einen Dosierinhalator zu entwickeln, bei dem der Patient klare und unmissverständliche Anweisungen darüber erhält, wie er zu atmen hart. Die Hauptaufgabe ist es, dass das Gerät eine exakt dosierte Menge von Aerosol produziert, deren Verbleib nachprüfbar ist. Deshalb soll kein Aerosol aus dem Gerät hinaus in die Umgebung geblasen werden kann, um die Dosierung möglichst genau einzuhalten und um unbeteiligte Personen vor dem Wirkstoff zu schützen. Das vollständige Einatmen jeder produzierten Teilmenge des Aerosols soll mittels einer zyklusgerechten Ansteuerung der Benutzerbefehlsausgabe gesichert werden.

Als Lösung schlägt die Erfindung einen Dosierinhalator vor, bei dem vor dem Zuluftrohr ein Aerosolfilter und ein Zuluftventil angeordnet sind und an den Aerosoldom ein Ausatmungsrohr angeschlossen ist, an dessen Ende ein Aerosolfilter und ein Ausatmungsventil angeordnet ist und Benutzerbefehle der Benutzerbefehlsausgabe den Betriebszuständen Ausatmen (Exhale) oder Einatmen (Inhale) oder Warten zugeordnet sind.

Dabei wird eine exakte Dosierung dadurch erreicht, dass bei normaler Raumtemperatur und in dem bekannten Volumen des Aerosol domes bei zuvor nachweislich aerosolfreier Innenluft, unabhängig von der exakten Betriebsdauer des Verneblers durch physikalische Sättigung der Innenluft eine ganz bestimmte Aerosolmenge mit hoher, reproduzierbarer Genauigkeit in jedem Betriebszyklus produziert wird. Für die Genauigkeit ist also nicht die Betriebsdauer des Verneblers wichtig, sondern dass die Innenluft zu Beginn eines jeden neuen Zyklus aerosolfrei ist und dass während der Aerosolproduktion der Vernebler so lange betrieben wird, bis der Zustand der Sättigung mit Sicherheit erreicht ist.

Die Produktion der exakt dosierten Aerosolmenge ist also gemäß dem aktuellen Stand der Technik sichergestellt.

Nach Abschluss der Produktion muss dann überwacht werden, dass das Aerosol bis zum nächsten Produktionszyklus vollständig aus d.em Innenraum unterhalb des Aerosoldomes verschwindet.

Die Grundidee der Erfindung ist es, dafür zu sorgen, dass diese Dosis des Wirkstoffes auch vollständig in die Lungenbläschen gelangt. Dazu dient dem erfindungsgemäßen Dosierinhalator eine Benutzerbefehlsausgabe, die in jedem Betriebszustand für den Benutzer des Gerätes erkennbar ist. Diese Befehisausgabe wird ebenso wie der Vernebler über die elektronische Steuerung gesteuert, sodass der zeitlich korrekte Ablauf gewährleistet ist.

Als alternative Ausführungsform für die Erzeugung des Aerosols kann zwischen dem Wirkflüssigkeitsbehältet und den Vernebler ein Übertragungsflüssigkeitsbehälter angeordnet werden. In dieser Variante wird der Wirkflüssigkeitsbehälter vorzugsweise kegelförmig ausgeführt. Die Wirkflüssigkeit wird in den auf seiner (abgerundeten) Spitze stehenden Hohlkegel eingefüllt. Die abgerundete Spitze des Kegels taucht in die Übertragungsflüssigkeit ein, welche vom Ultraschallschwinger zu Schwingungen angeregt wird, die über die Wandung des Wirkflüssigkeitsbehälters hinweg auf die Wirkflüssigkeit übertragen wird. Die Übertragungsflüssigkeit dient also als Kupplung für hochfrequente Schwingungen. Der entscheidende Vorzug dieser Anordnung ist, dass die Wirkflüssigkeft nur in einem sehr kleinen Bereich des Gerätes verbreitet wird und deshalb das Risiko der Kontaminierung mit unerwünschten Stoffen verringert wird.

Es hat sich bewährt, dass der untere Teil des Wirkflüssigkeitsbehälters aus einem dünnen und schwingungsfähigen Kunststoff hergestellt, nur für einen einzigen Inhalationsvorgang benutzt und danach entsorgt wird. Dadurch entfällt die Reinigung eines mehrfach zu benutzenden Behälters, was das Risiko des Einschleppens unerwünschter Bakterien in die Lunge drastisch reduziert.

Zur Überwachung des Vorhandenseins von Aerosol schlägt die Erfindung den Einbau eines Dichtesensors vor, der den Raum oberhalb des Wirkflüssigkeitsbehälters und unterhalb des Aerosoldomes überwacht. In seiner einfachsten Ausführungsform ist dieser Dichtesensor ein Infrarotsensor, der - mit einer gewissen Toleranz - erfassen kann, dass der überwachte Raum entweder frei von Aerosolen oder von einem Aerosol in der gewünschten Mindestkonzentration ausgefüllt ist. Dieser Infrarotsensor wird von der elektronischen Steuerung ausgewertet und für die Steuerung der Atembefehle genutzt. In einer Untervariante ist es denkbar, dass der Dichtesensor nicht nur die zwei Zustände "Aerosolfrei" und "Aerosol vorhanden" erfasst, sondern mit einer reproduzierbaren Genauigkeit auch Zwischenwerte erfassen kann. Bei dieser Untervariante ist es sinnvoll, dass durch Fehlnutzung nicht vollständig abtransportierte Aerosolmengen durch entsprechend erneuerte Atembefehle doch noch in die Lunge des Patienten befördert werden. Aber auch in seiner einfachsten Ausführung ist der Dichtesensor für die elektronische Steuerung die entscheidende Grundlage für die Überwachung der tatsächlichen Befolgung der Atemkommandos.

Für die Atemkommandos an den Benutzer ist jeder erfindungsgemäβe Dosierinhalator mit einer Benutzerbefehlsausgabe ausgestattet. Dabei sind die verschiedensten Varianten für diese Schnittstelle gemäß dem aktuellen Stand der Technik für Mensch- Maschine-, Schnittstellen realisierbar. Dazu gehören akustische Signale, wie einfache Töne, Melodien oder Sprache. Denkbar sind elektromechanische Signalgeber, wie z.B. die von Handys her bekannten Shaker, rotierende Elektroantriebe mit einem Unwuchtgewicht.

Die Erfindung bevorzugt jedoch optische Signale. Dazu zählen Leuchtdioden, Glühbirnen und andere selbstleuchtende Elemente, mechanisch klappbare Signalflächen ohne und mit Befeuchtung, sowie als bevorzugtes Element ein Display, z.B. als LCD (Liquid Cristal Display). Auf einem solchen Display können Symbole, andere graphische Darstellungen und Texte aufleuchten. Hier bieten sich vor allem englischsprachige Befehle an, da diese Sprache für elektronische Geräte der aktuelle, globale.Standard ist und weil in dieser Sprache vergleichsweise wenige Zeichen ausreichen.

Unabhängig von der gewählten Form der Benutzerbefehlsausgabe, sieht die Erfindung bereits in der einfachsten Version die folgenden fünf Befehle vor: Ausatmen, Einatmen, Warten. Zusätzlich sinnvoll sind "Ende" oder "Falschnutzung". Der Vergleich mit der englischen Übersetzung: Exhale, Innale, Wait, End, Fault, zeigt, welch geringe Anzahl von Buchstaben für die Befehlsübermittlung erforderlich sind.

Selbstverständlich sind jedoch auch, alle anderen Schriftarten und denkbaren symbolischen Darstellungen, die bekannt und üblich sind, für einen erfindungsgemäßen Dosierinhalator geeignet.

Als sinnvolle Bauform schlägt die Erfindung vor, dass vor dem Zuluftrohr ein Aerosolfilter und ein Zuluftventil angeordnet wird. Das Aersolfilter stellt sicher, dass auch in dem Zeitabschnitt, in dem das Zuluftventil noch nicht verschlossen ist, fast kein Aerosol nach außen bringt. Das ist vor allem in Interesse einer exakten Dosierung erforderlich. Ein weiterer Grund für den Einbau eines Filters ist, dass damit auch die Inhalation von Stoffen möglich wird, die zwar in kleiner Wirkung einen insgesamt heilsamen Effekt erzielen, jedoch in größeren Mengen für unbeteiligte Personen unangenehm oder sogar riskant sein können.

Als weitere, sinnvolle Ausstattung nennt die Erfindung eine Atmungserkennung im Zuluftrohr. Sie kann unterscheiden, ob im Zuluftrohr überhaupt eine Luftströmung herrscht und in welche Richtung diese fließt. In der Mindestkonfiguration wird die Luftmenge nur anhand eines einzigen Schwellwertes überwacht, der entweder unterschritten oder überschritten wird. In einer verfeinerten-Ausführung ist auch die Erfassung von Zwischenwerten möglich und sinnvoll.

Als eine weitere Einzelheit im Sinne einer verbesserten Erfüllung der Aufgabenstellung ist es auch denkbar, dass das ZuluftVentil nicht mehr als nur passives Element einen bestimmten Mindestluftstom benötigt, um sich zu schließen, sondern stattdessen durch einen eigenen, nur dem Zuluftventil zugeordneten Antrieb aktiv von der Steuerung aus geschlossen und geöffnet wird.

Gemäß dem aktuellen Stand der Technik für Inhalatoren kann auch ein erfindungsgemäßer Dosierinhalator an seinem Abluftrohr mit einem Mundstück, einem Nasenstück oder einer Gesichtsmaske erweitert werden. Denkbar ist auch, dass - z.B. für bettlägrige Patienten - noch ein kleiner Verlängerungsschlauch dazwischen eingefügt wird, damit bei beliebiger Ausrichtung des Gesichtes vom Patienten die Funktionsbereiche des Dosierinhalators in der zu bevorzugenden vertikalen Ausrichtung bleiben. In diesem Fall ist darauf zu achten, dass der Benutzer trotzdem die Befehle der Benutzerbefehlsausgabe wahrnehmen kann. Ein optisches Display darf also nicht verdeckt werden oder aus dem Blickwinkel des patienten geraten. Widrigenfalls muss für diese Anwendungsfälle ein klappbares Display eingebaut werden.

Damit der Patient als Komfortdetail zum Ausatmen nicht seinen Mund von Gerät lösen muss und damit auch das Ausatmen überwacht werden kann, ist ein erfindungsgemäßer Dosierinhalator mit einem Ausatmungsrohr versehen, das z.B. am Aerosoldom angeschlossen werden kann.

Das Ausatmungsfilter sollte entsprechend dem Zuluftrohr ebenfalls am Ende über einen Aerosolfilter und ein Ausatmungsventil verfügen.

Die Erfindung bevorzugt, dass das Ausatmungsrohr ebenfalls an die Atmungserkennung angeschlossen ist. Denkbar ist, dass auch das Ventil im Ausatmungsrohr einen eigenen Antrieb erhält, der von der elektronischen Steuerung aktiviert werden dann. Ähnlich wie bei einem fremdgetriebehen Ventil im Zuluftrohr wird Damit auch im Ausatmungsröhr schon vor dem Auftreten einer Luftströmung in eine - in dieser Phase - nicht erwünschte - Richtung, das Ventil verschlossen, sodass ein unerwünschtes Ein- und Austreten von Aerosol durch das Ausatmungsrohr vollständig vermieden werden kann.

Ein erfindungsgemäße Dosierinhalator, der mit allen bis hier beschriebenen Zusatzausstattungen ausgerüstet ist, sichert das vollständige Einatmen jeder produzierten Teilmenge des Aerosols in der ersten, aktiven Ebene der Steuerung über die Benutzerbefehlsausgabe, die zyklusgerecht angesteuert wird. Für eine sachgerechte Benutzung des Dosierinhalators muss der Patient also nur den Dosierinhalator mit den jeweils vorgeschriebenen Wirkstoffen in der vom Arzt vorgesehenen Menge bestücken und sicherstellen, dass das Gerät gereinigt ist und mit einer ausreichenden Menge von übertragungsflüssigkeit versehen ist.

Nach diesen Vorarbeiten reicht das Aktivieren des Dosierinhalators durch einen einzigen, simplen Startknopf. Daraufhin beginnt der Dosierinhalator mit der Produktion von Aerosol und signalisiert dem Benutzer derweil "Ausatmen". Wenn die Aerosolproduktion abgeschlossen ist, schaltet die elektronische Steuerung den Vernebler aus und signalisiert dem Patienten über die Benutzerbefehlsausgabe "Einatmen". Sobald die Einatmungszeit verstrichen ist, schaltet der Dosisrinhalator in die Phase "Warten" um, während derer die Luftströmung im Innenraum abklingt und evtl. noch schwebende, große Aerosoltropfen wieder in den Wirkflüssigkeitsbehälter zurückfallen. Nach dieser Wartezeit beginnt eine neue Phase des Ausatmens, während derer die nächste Aerosolproduktion anläuft. Damit beginnt der nächste Zyklus.

Ebenso wie bei allen anderen MMI (Man-Machine-Interfaces) ist auch die Benutzerbefehlsausgabe des erfindungsgemäßen Dosierinhalators letzten Endes darauf angewiesen, dass der Nutzer die gegebenen Befehle auch tatsächlich befolgt. Um dieses zu erleichtern, sind sie - wie zuvor beschrieben - klar und eindeutig formuliert und leuchten in einer sinnvollen Reihenfolge auf. Wenn der Benutzer diese eindeutigen Befehle befolgt, ist auch eine einwandfreie Funktion eines jeden Schrittes sichergestellt. In der Praxis ist es jedoch vorstellbar, dass der Benutzer die Befehle doch nicht vollständig ausführt, sondern durch mangelnde Konzentration und/oder Schwächung und/oder Demenzen und/oder aus anderen Gründen die Befehle nur teilweise oder gar nicht befolgt oder sogar das Gegenteil dessen tut, was die Benutzerbefehlsausgabe vorgibt.

Für diese Fälle bietet ein erfindungsgemäßer Dosierinhalator als zusätzliche Ausstattung, dass mittels eines Dichtesensors in der zweiten, passiven Ebene der Steuerung das Vorhandensein bzw. Fehlen von Aerosol überwacht wird, sowie im Zuluftrohr und im Ausatmungsrohr die Luftrichtung und die Luftmenge erkannt wird. Zusätzlich sind in dieser zweiten Überwachungsebene weitere Verknüpfungen und Kontrollen möglich, mit der die Funktionssicherheit des Dosierinhalators auch bei Fehlbedienung weiter verbessert wird. So ist es sinnvoll, dass beim Vorhandensein eines angetriebenen Ausatmungsventiles und/oder Zuluftventiles das Befehlszeichen für "Einatmen" erst nach dem Öffnen des Zuluftventiles und/oder dem Verschließen des angetriebenen Ausatmungsventiles ausgebbar ist. Entsprechend sollte das Befehlszeichen für "Ausatmen" erst nach einem Verschließen des angetriebenen Zuluftventiles und/oder dem Öffnen des angetriebenen Ausatmungsventiles ausgegeben werden.

Wie geschildert, besteht ein jeder Atemzyklus bei der Verwendung eines erfindungsgemäßen Dosierinhalators aus der Phase 1 "Aerosolaufbereitung", der Phase 2 "Einatmen" und der Phase 3 "Warten" Für eine noch weiter verbesserte Überwachung der tatsächlichen Befolgung dieser Befehle an den Benutzer sind weitere regelungstechnische Verknüpfungen der elektronischen Steuerung mit den Sensoren und der Benutzerbefehlsausgabe sinnvoll.

So ist es eine zusätzliche Sicherung, dass in der ersten Phase "Aerosolaufbereitung" das Erreichen der gewünschten Aerosotmenge durch den Dichtesensor laufend überprüft wird und bei Nichterreichen der Vernebler nochmals aktiviert ist, solange bis die gewünschte Aerosoldichte für den Dichtesensor erkennbar ist.

In einer weiteren Verfeinerung der Steuerung kann die Anzahl der möglichen Versuche begrenzt werden und nach Überschreiten dieser Anzahl eine Fehlermeldung ausgegeben werden. Dahinter steht der Gedanke, dass im Normalfall bereits nach einem einzigen Schweingungszyklus die gewünschte Aerosolmenge produziert und voranden ist. Wenn auch nach mehreren, vergeblichen Versuchen der Dichtesensor immer noch keine Aerosolmenge als vorhanden meldet, ist entweder der Vernebler defekt, die Flüssigkeit fehlt, oder der Dichtesensor ist nicht mehr funktionsbereit.

Als weiteres Komfortdetail in der Ansteuerung der Benutzerbefehlsausgabe beschreibt die Erfindung das Problem, dass zwar das Befehlszeichen für "Ausatmen" aktiviert ist, aber die Atmungserkennung am Ausatmungsrohr passiv ist, was darauf hin deutet, dass der Patient in diesem Moment nicht den gegebenen Befehl "Ausatmen" befolgt. Als Reaktion kann die elektronische Steuerung das Befehlszeichen für "Ausatmen" in der Benutzerbefehlsausgabe verstärken, z.B. durch Blinken der ersten Anzeige oder das Aktivieren einer weiteren Befehlsanzeige, wie z.B. einer Sprachausgabe.

In Entsprechung zum vorgenannten Problem des Unterlassens von phasengerechtem Ausatmen kann auch in der (wichtigen) Einatmungsphase bei einem Ausbleiben der Atemerkennung der Befehl für "Einatmen" ,wie zuvor geschildert, verstärkt werden.

Bei beiden Befehlsverstärkungen sollte das (verstärkte) Signal nach Überschreitung der jeweils vorgesehenen Höchstzeit abgeschaltet werden. Wenn z.B. in der Phase "Einatmen" die Atmungserkennung am Zuluftrohr nur zeitweise oder gar nicht aktiv ist und wenn der Dichtesensor immer noch das Vorhandensein von Aerosol meldet, sollte trotzdem die elektronische Steuerung auf den Befehl "Ausatmen" umschalten. Wenn nach dem Ende der vorgesehenen Zeit für das Ausatmen der Dichtesensor noch immer das Vorhandensein von Aerosol meldet und wenn die maximale "Lebensdauer" eines Aerosols noch nicht überschritten ist, sollte anschließend erneut der Befehl zum Einatmen erteilt werden, damit die verbliebene Restaerosolmenge vollständig absorbiert wird.

Es ist einer der wesentlichen Vorteile der erfindungsgemäßen Dosierinhalatoren, dass bei Nichterfolgen einer Inhalation die elektronische Steuerung die produzierte Aerosolmenge nicht als bereits inhaliert einstuft, sondern davon ausgeht, dass sie wieder zurück in den Wirkflüssigkeitsbehälter gelangt ist. Dementsprechend wird die elektronische Steuerung wenigstens einen zusätzlichen Zyklus starten, um sicher zu stellen, dass am Ende des Vorganges die gewünschte Dosiermenge dennoch vom Patienten tatsächlich aufgenommen worten ist.

Damit kann die elektronische Steuerung berücksichtigen, dass das Aerosol nur für eine bestimmte Zeit stabil ist und danach die Tröpfchen entweder direkt in den Wirkflüssigkeitsbehälter zurückgelangt sind oder über Kondensierung an den Wandungen und Zurücklaufen in den Behälter wieder dorthin gekommen sind.

Im Folgenden sollen weitere Einzelheiten und Merkmale der Erfindung anhand von Beispielen näher erläutert werden. Diese sollen die Erfindung jedoch nicht einschränken, sondern nur erläutern. Es zeigt in schematischer Darstellung:
- **Figur 1**: Aufsicht auf einen Dosierinhalator mit zwei gegeneinander versetzten Schnittebenen für Figur 2 und 3
- **Figur 2**: Schrägbild eines Dosierinhalators mit Schnitt durch Aerosoldom und Ausatmungsrohr in der Phase "Ausatmen"
- **Figur 3**: Schrägbild eines Dosierinhalators mit Schnitt durch Aerosoldom und Ausatmungsrohr in der Phase "Einatmen"

Die Figuren zeigen im Einzelnen:

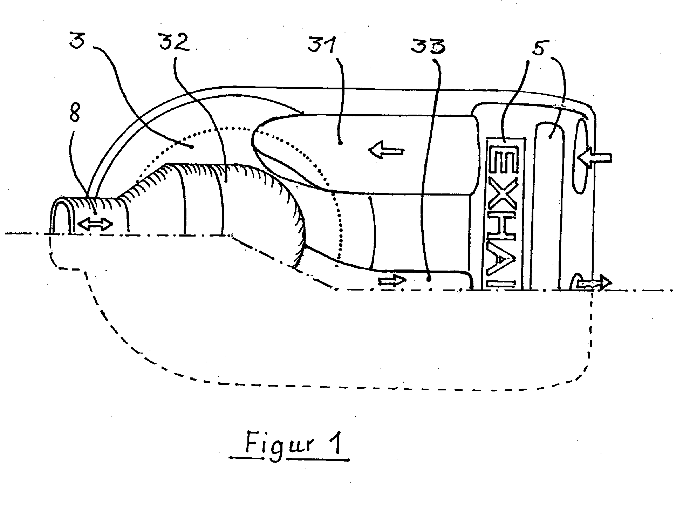

**Figur 1** gibt die Aussicht auf einen erfindungsgemäßen Dosierinhalator wieder. In der linken Hälfte ist der Aerosoldom 3 mit einer gepunkteten Linie eingezeichnet. Gepunktet deshalb, weil er durch das Abluftrohr 32 und die daran anschließende Kuppel, welche konzentrisch zu dem Aerosoldom 3 angeordnet ist, verdeckt wird.

Ebenfalls konzentrisch zum Aerosoldom 3 ist das Abluftrohr 32 angeordnet. In der Aufsicht des Gerätes in Figur 1 wird an der linken Seite das seitlich vom Abluftrohr 32 ausgehende Mundstück 8 sichtbar. In Figur 1 kann nachverfolgt werden, wie eingeatmete Luft durch die oben rechts dargestellte Öffnung eintritt, unterhalb der Benutzerbefehlsausgabe 5 hindurch in das Zuluftrohr 31 eintritt und von dort in die Kuppel über dem Aerosoldom 3 gelangt. Die Luftrichtung ist durch zwei Pfeile markiert.

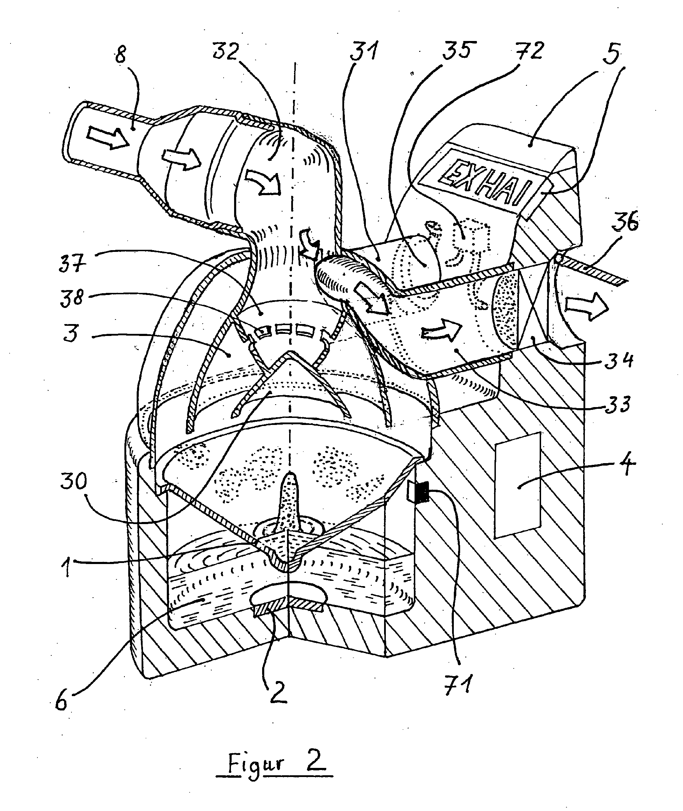

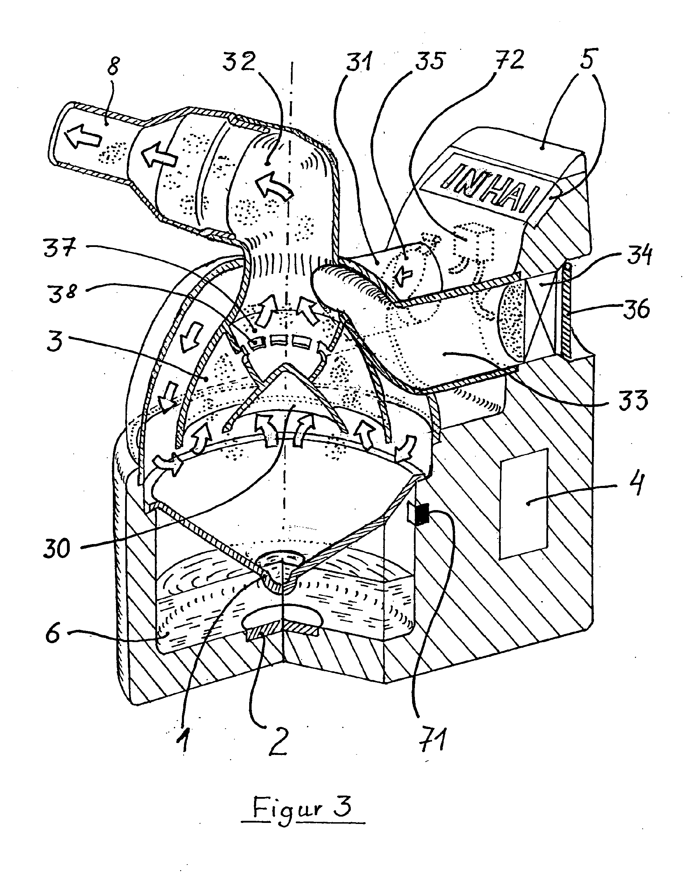

Im Zustand "Ausatmen" bewegt sich die Luft im Mundstück 8 in der anderen Richtung, was durch einen Doppelpfeil markiert ist. Die ausgeatmete Luft tritt dann durch das Ausatmungsrohr 33 wieder aus. Vom Ausatmungsrohr 33 ist nur ein Teil gezeichnet; der Rest ist in der Darstellung abgeschnitten. Ebenso ist auch das Mundstück 8 nur zur Hälfte dargestellt. In Figur 1 wird die zweimal geknickte Schnittebene der Figuren 2 und 3 durch eine Strich-Punkt-Strich-Linie dargestellt.

Die Silhouette des vollständigen Gerätes ist im unteren Bereich von Figur 1 durch eine gestrichelte Linie eingetragen.

**In** **Figur 2** ist der in Figur 1 in der Aufsicht dargestellte Dosierinhalator als Schrägbild dargestellt und zwar mit einem Schnitt in der in Figur 1 definierten, zweimal geknickten Schnittebene. Dadurch wird der Einblick in den Mittelpunkt des Wirkflüssigkeitsbehälters 1 mit dem Sprudel möglich und ebenso ein Schnitt durch das Ausatmungrohr 33.

In Figur 2 ist auf dem Boden des Übertragungsflüssigkeitsbehälters 6 der Vernebler 2, ein Ultraschallschwinger, erkennbar. Es wird nachvollziehbar, wie sich die Schwingungen des Verneblers 2 über die Übertragungsflüssigkeit im Übertragungsflüssigkeitsbehälter 6 und über den - in diesem Beispiel kegelförmigen - Wirkflüssigkeitsbehälter 1 auf die Wirkflüssigkeit übertragen.

In Figur 2 ist die erste Phase des Zyklusses dargestellt, nämlich die Aerosolaufbereitung. In dieser Phase schwingt der Vernebler 2 und überträgt die Schwingungen auf die Übertragungsflüssigkeit und von fort über den Wirkflüssigkeitsbehälter auf die Wirkflüssigkeit selbst. Diese bildet dadurch den in Figur 2 erkennbaren Sprudel aus, welcher die Aerosolwolken absondert, die in Figur 2 als gepunktete Bereiche innerhalb des Wirkflüssigkeitsbehälters 1 zu erkennen sind.

Während der Aerosolaufbereitung soll der Patient ausatmen. Das wird durch den Befehl "Exhale" in der Benutzerbefehlsausgabe 5 angeordnet. In dieser Phase muss der Patient seinen Mund nicht vom Mundstück 8 lösen, sondern kann normal weiter atmen, was der durch Pfeile gekennzeichnete Luftstrom darstellt. Die Luft strömt aus dem Mundstück 8 in das Abluftrohr 32 und von dort durch die Schlitze 38 in den Aerosoldom 3 und in das Ausatmungsrohr 33. Die Luft tritt durch den Aerosolfilter 34 hindurch, öffnet das Ausatmungsventil 36 und tritt dann in die Umgebungsluft aus. In Figur 2 ist erkennbar, wie die Ausatmungsluft durch ihre Strömung das Ausatmungsventil 36 geöffnet hat.

In Figur 2 wird nachvollziehbar, dass in der Phase des Ausatmens kein Aerosol aus dem Bereich unterhalb des Aerosoldomes 3 austreten kann, weil sich der durch Ausatmen etwas erhöhte Luftdruck auch bis in das Zuluftrohr 31 fortpflanzt und dort das Zuluftventil 35 verschließt, sodass nur der Weg durch das Ausatmungsrohr 33 bleibt.

In Figur 2 ist gepunktet die Atmungserkennung 72 eingezeichnet, die mit einem kleinen Schlauch an das Ausatmungsrohr 33 angeschlossen ist. Die Atmungserkennung 72 erkennt, dass in diesem Zustand Luft durch das Ausatmungsrohr strömt.

In Figur 2 wird plausibel, dass der Dichtesensor 71 als Infrarotsensor durch die Wandung des Wirkflüssigkeitsbehälters 1 hindurch das Vorhandenensein der - hier gepunktet dargestellten - Aerosolwolken erfassen kann.

**In** **Figur 3** ist der gleiche Schnitt durch einen dreidimensional gezeichneten Dosierinhalator wiedergegeben wie in Figur 2, jedoch im Zustand "Einatmen" (Inhale).

In Figur 3 sind die Aerosolwolken jetzt innerhalb des Hohlkegelstumpfes der Prallplatte 30 erkennbar. Zwei Pfeile zeigen an, wie sich die Aerosolwolken dort verwirbeln.

Ein weiterer Pfeil im Zuluftrohr 31 zeigt die Richtung der eintretenden Luft an. Erkennbar ist, dass das Zuluftventil 35 in Zustand "Einatmen" geöffnet ist. Das daneben gelegene Ausatmungsventil. 36 ist in dieser Phase verschlossen und in das Gerät zurückgeschwenkt.

In Figur 3 kann der weitere Verlauf der eingeatmeten Luft verfolgt werden: Aus dem Zuluftrohr 31 tritt die Luft in eine Kuppel über dem Aerosoldom 3 ein. Zwei Pfeile im linken, angeschnittenen Bereich dieser Kuppel zeigen den Verlauf der Luftrichtung. Die Luft strömt dann weiter zur Unterkante des Aerosoldomes 3 und tritt dort in den Aerosoldom 3 ein. Dabei reißt sie auch die Aerosolwolken mit, die sich unterhalb der Prallplatte 30 befinden. Von dem Sog der Einatmung werden die Aerosolwolken innerhalb des Aerosoldomes 3 wieder nach oben strömen und treten durch die Schlitze 38 in der hohlkegelförmigen Halterung 37 hindurch in das Abluftrohr 32 ein. Dort sind die - gepunktet dargestellten - Aerosolwolken erkennbar, die weiter in das Mundstück 8 strömen und von dort in die Lunge des Patienten.

In Figur 3 wird nachvollziehbar, dass im dargestellten Schluss - Teil der Phase "Einatmen" der Dichtesensor 71 nicht mehr das Vorhandensein von Aerosol im Wirkflüssigkeitsbehälter 1 melden kann, da sämtliche Aerosolwolken bereits ihren Weg aus dem Wirkflüssigkeitsbehälter 1 herausgefunden haben.

Die elektronische Steuerung 4 ist in den Figuren 2 und 3 nur schematisch eingezeichnet. Ihre funktionelle Verbindung zu dem Vernebler 2, dem Dichtesensor 71 und der Atmungserkennung 72 ist nicht eingezeichnet.

## Patentansprüche

1. Dosierinhalator, bestehend aus
- einem Wirkflüssigkeitsbehälter 1, in dem sich eine Flüssigkeit mit einem darin gelösten Wirkstoff befindet sowie
- einem Aerosoldom 3, in welchem
- eine Prallplatte 30 angeordnet ist und an welchem
- ein Zuluftrohr 31 sowie
- ein Abluftrohr 32 angeschlossen ist
- einem Vernebler 2, durch den Flüssigkeit in ein Aerosol umwandelbar und in den Aerosoldom 3 einbringbar ist, sowie
- einer elektronischen Steuerung 4, die den Vernebler 2 und die Benutzerbefehlsausgabe 5 ansteuert.
wobei
- der Vernebler 2 intermittierend betreibbar ist und
- eine Benutzerbefehlsausgabe 5 für den Benutzer bemerkbar ist und
- die Erzeugung der innerhalb und unterhalb des Aerosoldomes 3 befindlichen Aerosolmenge von deren Inhalation zeitlich trennbar ist
**dadurch gekennzeichnet, dass**
vor dem Zuluftrohr 31 ein Aerosolfilter 34 und ein Zuluftventil 35 angeordnet sind und
an den Aerosoldom 3 ein Ausatmungsrohr 33 angeschlossen ist, an dessen Ende ein Aerosolfilter 34 und ein Ausatmungsventil 36 angeordnet ist und
Benutzerbefehle der Benutzerbefehlsausgabe 5 den Betriebszuständen
- Ausatmen (Exhale) oder
- Einatmen (Inhale) oder
- Warten
zugeordnet sind. -

2. Dosierinhalator nach dem vorhergehenden Anspruch, **dadurch gekennzeichnet, dass** der Wirkflüssigkeitsbehälter 1 so angeordnet ist, dass er teilweise in einen Übertragungsflüssigkleitsbehälter 6 eintaucht, in dem der Vernebler 2 angeordnet und auf den Wirkflüssigkeitsbehälter 1 ausgerichtet ist, wobei der eingetauchte Bereich des Wirkflüssigkeitsbehälters 1 eine dünne, elastische Wandung aufweist.

3. Dosierinhalator nach einem der vorhergehenden Ansprüche, dadurch gekenntzeichnet, dass die Dichte des Aerosols innerhalb und unterhalb des Aerosoldomes 3 durch einen Dichtesensor 71 messbar ist.

4. Dosierinhalator nach Anspruch 3, **dadurch gekennzeichnet, dass** der Dichtesensor 71 ein Infrarotsensor ist.

5. Dosierinhalator nach seinem der vorhergehenden Ansprüche **dadurch gekennzeichnet, dass** von der Benutzerbefehlausgabe 5
- Töne und/oder
- Symbole auf einem Display und/oder
- Text auf einem Display und/oder
- Sprache und/oder
- aus- und einschaltbare, optische Anzeigeelemente wie LED's Glühbirnen und/oder klappbare Signalflächen
aktivierbar sind.

6. Dosierinhalator nach einem der vorhergehenden Ansprüche **dadurch gekennzeichnet, dass** Benutzerbefehle den Betriebszuständen
- Ende oder
- Falschnutzung
zugeordnet sind

7. Dosierinhalator nach einem der vorhergehenden Ansprüche, **dadurch gekennzeichnet, dass**
an das Zuluftrohr 31 eine Atmungserkennung 72 angeschlossen ist.

8. Dosierinhalator nach einem der vorhergehenden Ansprüche, **dadurch gekennzeichnet, dass**
das Zuluftventil 35 mittels eines Antriebes von der elektronischen Steuerung 4 verschließbar oder offenbar ist.

9. Dosierinhalator nach einem der vorhergehenden Ansprüche, **dadurch gekennzeichnet, dass** an das Abluftrohr 32 ein Mundstück 8 oder ein Nasenstück oder eine Gesichtsmaske anschließbar sind.

10. Dosierinhalator nach einem der vorhergehenden Ansprüche, **dadurch gekennzeichnet, dass**
an das Ausatmungsrohr 33 eine Atmungserkennung 72 angeschlossen ist.

11. Dosierinhalator einem der vorhergehenden Ansprüche, **dadurch gekennzeichnet, dass**
das Ausatmungsventil 36 mittels eines Antriebes von der elektronischein Steuerung 4 verschließbar oder öffenbar ist.

12. Dosierinhalator nach einem der vorhergehenden Ansprüche **dadurch gekennzeichnet, dass** die Zeitspanne, während der ein Aerosol ausreichend stabil ist, in der Steuerung 4 einstellbar ist.

13. Dosierinhalator nach einem der vorhergehenden Ansprüche **dadurch gekennzeichnet, dass** das Befehlszeichen für "Einatmen" (INHALE) erst nach dem Öffnen des angetriebenen Zuluftventiles 35 und/oder dem Verschließen des angetriebenen Ausatmungsventiles 36 aushebbar ist.

14. Dosierinhalator nach einem der vorhergehenden Ansprüche **dadurch gekennzeichnet, dass** das Befehlszeichen für "Ausatmen" (EXHALE) erst nach einem Verschließen des angetriebenen Zuluftventiles 35 und/oder dem Öffnen des angetriebenen Ausatmungsventiles 36 ausgebbar ist.

15. Dosierinhalator nach einem der vorhergehenden Ansprüche **dadurch gekennzeichnet, dass** von der Steuerung 4 derartige Befehle abgebbar sind, dass
in der ersten Phase "Aerosolaufbereitung" der Vernebler 2 die gewünschte Aerosolmenge produziert und in den Aerosoldom 3 einbringt und die Benutzerbefehlsausgabe 5 das Befehlszeichen für "Ausatmen" (EXHALE) ausgibt und
in der zweiten Phase "Einatmen" die Benutzerbefehlsausgabe 5 das Befehlszeichen für "Einatmen" ausgibt und
in der dritten Phase "Warten" die Benutzerbefehlsausgabe 5 das Befehlszeichen für "Warten" (WAIT) ausgibt und
danach ein weiterer Zyklus mit den gleichen drei Phasen abläuft

16. Dosierinhalators nach Anspruch 15, **dadurch gekennzeichnet, dass** von der Steuerung 4 derartige Befehle abgebbar sind, dass in der ersten Phase "Aerosolaufbereitung" das Erreichen der gewünschten Aerosolmenge durch den Dichtesensor 71 laufend überprüft wird und bei Nichterreichen der Vernebler 2 nochmals aktiviert wird, bis die gewünschte Aerosolmenge produziert ist.

17. Dosierinhalator nach Anspruch 15, **dadurch gekennzeichnet, dass**, wenn in der ersten Phase die Benutzerbefehlsausgabe 5 das Befehlszeichen für ,Ausatmen" aktiviert hat, aber die Atmungserkennung 72 am Ausatmungsrohr 33 nicht aktiv ist, die elektronische Steuerung 4 in der Benutzerbefehlsausgabe 5 das Befehlszeichen für "Ausatmen" verstärkt ist, z. B. durch Blinken einer optischen Anzeige und/oder zusätzliches Aktivieren einer weiteren Befehlsanzeige wie z.B. eines akustischen Signals

18. Dosierinhalator nach Anspruch 15, **dadurch gekennzeichnet, dass** in der zweiten Phase "Einatmen" bei fehlender Aktivierung der Atmungserkennung 72 die elektronische Steuerung 4 das Befehlszeichen für "Einatmen" verstärkt, z. B. durch Blinken der optischen Anzeige und/oder zusätzliches Aktivieren einer weiteren Befehlsanzeige wie z.B. eines akustischen Signals und
das Befehlszeichen für "Einatmen" nach einer bestimmten, einstellbaren Einatmungszeitgrenze abschaltet.

19. Dosierinhalator nach Anspruch 18, **dadurch gekennzeichnet, dass** von der Steuerung 4 derartige Befehle abgebbar sind, dass
in der zweiten Phase Einatmen", wenn die Atmungserkennung 72 am Zuluftrohr 31 nur zeitweise oder gar nicht aktiv ist und wenn der Dichtesensor 71 immer noch das Vorhandensein von Aerosol meldet, die elektronische Steuerung 4 in der Benutzerbefehlsausgabe 5 das Befehlszeichen für "Einatmen" nach Überschreiten der gemäß Anspruch 12 eingestellten Maximalzeit für die Stabilität des Aerosols abschaltet.

20. Dosierinhalators nach Anspruch 19, **dadurch gekennzeichnet, dass** nach Überschreitung der Maximalzeit für die Stabilität des Aerosols die elektronische Steuerung 4 die nicht inhalierte Menge des Aerosols bei der Berechnung der bisher inhalierten Gesamtmenge nicht berücksichtigt.

## Claims

1. Dosing Inhaler, consisting of
- an active liquid container 1, which contains a liquid having an active substance dissolved therein, and
- an aerosol dome 3, in which
- a baffle plate 30 is arranged, and to which
- a supply-air tube 31 as well as
- an exhaust-air tube 32 are connected,
- an atomizer 2, by means of which liquid can be transformed into an aerosol and introduced into the aerosol dome 3, as well as
- an electronic controller 4, which actuates the atomizer 2 and the user-command output 5
wherein
- the atomizer 2 can be operated intermittently, and
- a user-command output 5 is noticeable for the user, and
- the generation of the aerosol volume within and below the aerosol dome 3 can be separated in time from the inhalation thereof, **characterised in that** an aerosol filter 34 and a supply-air valve 35 are disposed upstream of the supply-air tube 31 and
an exhalation tube 33, at the end of which an aerosol filter 34 and an exhalation valve 36 are disposed, is connected to the aerosol dome 3, and user commands of the user command output 5 are assigned to the operating states
- exhale or
- inhale or
- wait.

2. Dosing inhaler according to the preceding claim, **characterised in that** the active liquid container 1 is arranged such that it is partially immersed in a carrier liquid container 6, in which the atomizer 2 is arranged ,and is aligned with respect to the active-liquid container 1, the immersed region of the active-liquid container 1 having a thin, elastic wall.

3. Dosing inhaler according to one of the preceding claims, **characterised in that** the density of the aerosol within and below the aerosol dome 3 can be measured by means of a density sensor 71.

4. Dosing inhaler according to claim 3, **characterised in that** the density sensor 71 is an infrared sensor.

5. Dosing inhaler according to one of the preceding claims, **characterised in that**
- sounds and/or
- symbols on a display and/or
- text on a display and/or
- speech and/or
- optical display elements that can be switched on and off, such as LEDs, incandescent lamps and/or hinged signal faces, can be activated by the user-command output 5.

6. Dosing inhaler according to one of the preceding claims, **characterised in that** user commands are assigned to the operating states
- end or
- incorrect use.

7. Dosing inhaler according to one of the preceding claims, **characterised in that** a respiration detector 72 is connected to the supply air tube 31.

8. Dosing inhaler according to one of the preceding claims, **characterised in that** the supply-air valve 35 can be closed or opened by the electronic controller 4 by means of a drive.

9. Dosing inhaler according to one of the preceding claims, **characterised in that** a mouthpiece 8 or a nose piece or a face mask can be connected to the exhaust-air tube 32.

10. Dosing inhaler according to one of the preceding claims, **characterised in that** a respiration detector 72 is connected to the exhalation tube 33.

11. Dosing inhaler according to one of the preceding claims, **characterised in that** the exhalation valve 36 can be closed or opened by the electronic controller 4 by means of a drive.

12. Dosing inhaler according to one of the preceding claims, **characterised in that** the time span during which an aerosol is sufficiently stable can be set in the controller 4.

13. Dosing inhaler according to one of the preceding claims, **characterised in that** the command signal for inhalation ("INHALE") can only be issued after the opening of the driven supply-air valve 35 and/or after the closing of the driven exhalation valve 36.

14. Dosing inhaler according to one of the preceding claims, **characterised in that** the command signal for exhalation ("EXHALE") can only be issued after the closing of the driven supply-air valve 35 and/or after the opening of the driven exhalation valve 36.

15. Dosing inhaler according to one of the preceding claims, **characterised in that**
the commands can be issued by the controller 4 such that in the first phase "aerosol preparation", the atomizer 2 produces the desired aerosol volume and introduces it into the aerosol dome 3, and the user-command output 5 issues the command signal for exhalation (EXHALE) and
in the second phase "inhalation" the user-command output 5 issues the command signal for "inhalation" and
in the third phase "wait" the user-command output 5 issues the command for "waiting" (WAIT), and then a further cycle with the same three phases is executed.

16. Dosing inhaler according to claim 15, **characterised in that** commands can be issued by the controller 4 such that, in the first phase, "aerosol preparation", the reaching of the desired aerosol volume by the density sensor 71 is continually checked and, if it is not reached, the atomizer 2 is activated again, until the desired aerosol volume has been produced.

17. Dosing inhaler according to claim 15, **characterised in that** if the user command output 5 has activated the command signal for "exhalation" in the first phase, but the respiration detector 72 on the exhalation tube 33 is not active, the electronic controller 4 in the user command output 5 reinforces the command signal for "exhalation", e.g. by flashing an optical indicator and/or by additional activation of a further command display, such as an acoustic signal.

18. Dosing inhaler according to claim 15, **characterised in that**, in the second phase "inhalation", if the respiration detector 72 is not activated, the electronic controller 4 reinforces the command signal for "inhalation", for example by flashing the optical indicator and/or by additional activation of a further command display, such as an acoustic signal and the command signal for "inhalation" is shut off after a particular, adjustable inhalation time limit.

19. Dosing inhaler according to claim 18, **characterised in that** commands can be issued by the controller 4 such that
in the second phase "inhalation", if the respiration detector 72 on the supply-air tube 31 is only partially or not at all active, and if the density sensor 71 still reports the presence of aerosol, the electronic controller 4 in the user-command output 5 switches off the command signal for "inhalation" after the maximum time for the stability of the aerosol, as set according to claim 12, has been exceeded.

20. Dosing inhaler according to claim 19, **characterised in that**, after the maximum time for the stability of the aerosol has been exceeded, the electronic controller 4 does not take into account the non-inhaled volume of the aerosol in calculating the total amount that has been inhaled.

## Revendications

1. Inhalateur doseur consistant en
• un réservoir de liquide actif 1 dans lequel se trouve un liquide avec un agent actif ainsi
• qu'un dôme aérosol 3 dans lequel
• une plaque d'impact 30 est disposée et auquel
• un tuyau d'alimentation en air frais 31 et
• un tuyau d'évacuation de l'air vicié 32 sont raccordés
• un vaporisateur 2, à l'aide duquel le liquide peut être transformé en aérosol et pouvant être apporté dans le dôme aérosol 3, ainsi
• qu'une commande électronique 4, qui commande le vaporisateur 2 et le transmetteur d'ordre d'utilisateur 5
sachant que
• le vaporisateur 2 peut fonctionner de façon intermittente et
• qu'on peut remarquer un transmetteur d'ordre 5 pour l'utilisateur,
• la génération de la quantité d'aérosol qui se trouve à l'intérieur et en dessous du dôme aérosol 3 peut être séparée de leur inhalation dans le temps
**caractérisé par le fait que**
qu'un inhalateur doseur aérosol 34 et la vanne d'alimentation en air frais 35 sont disposés devant le tuyau d'alimentation en air frais 31 et qu'un tuyau d'expiration 33 est raccordé au dôme aérosol 3, à l'extrémité duquel sont disposés un inhalateur doseur aérosol 34 et une vanne d'expiration 36,
les ordres d'utilisateur du transmetteur d'ordre d'utilisateur 5 étant attribués aux états de fonctionnement
• expiration (EXHALE) ou
• inspiration (INHALE) ou
• attente.

2. Inhalateur doseur selon la revendication précédente, **caractérisé par le fait que** le réservoir de liquide actif 1 est disposé de façon à ce plonger en partie dans un réservoir de liquide de transmission 6, dans lequel le vaporisateur 2 est disposé et sur lequel le réservoir de liquide actif 1 est orienté, sachant que la partie immergée du réservoir de liquide actif 1 présente une paroi élastique mince.

3. Inhalateur doseur selon une des revendications précédentes, **caractérisé par le fait que** la densité de l'aérosol peut être mesurée à l'intérieur de et au-dessous du dôme aérosol 3 par un capteur de densité 71.

4. Inhalateur doseur selon la revendication 3, **caractérisé par le fait que** le capteur de densité 71 est un capteur à infrarouge.

5. Inhalateur doseur selon une des revendications précédentes,
**caractérisé par le fait que** le transmetteur d'ordre d'utilisateur peut activer
• des sons et/ou
• des symboles sur un écran et/ou
• un texte sur un écran et/ou
• la langue et/ou
• des éléments d'affichage optique, tels que des ampoules de LED et/ou des surfaces de signal rabattables.

6. Inhalateur doseur selon une des revendications précédentes,
**caractérisé par le fait que** les ordres d'utilisateur sont attribués aux états de fonctionnement
• fin ou
• utilisation erronée.

7. Inhalateur doseur selon une des revendications précédentes,
**caractérisé par le fait qu'**une reconnaissance de respiration 72 est raccordée au tuyau d'alimentation en air frais 31.

8. Inhalateur doseur selon une des revendications précédentes,
**caractérisé par le fait que** la vanne d'alimentation en air frais 35 peut être fermée ou ouverte par la commande électronique 4, au moyen d'un entraînement.

9. Inhalateur doseur selon une des revendications précédentes,
**caractérisé par le fait qu'**un embout buccal 8 ou un embout nasal ou un masque facial peut être raccordé au tuyau d'évacuation de l'air vicié 32.

10. Inhalateur doseur selon une des revendications précédentes,
**caractérisé par le fait qu'**une reconnaissance de respiration 72 est raccordée au tuyau d'expiration 33.

11. Inhalateur doseur selon une des revendications précédentes,
**caractérisé par le fait que** la vanne d'expiration 36 peut être fermée ou ouverte par la commande électronique 4, au moyen d'un entraînement.

12. Inhalateur doseur selon une des revendications précédentes,
**caractérisé par le fait que** le laps de temps pendant lequel un aérosol est suffisamment stable peut être réglé dans la commande 4.

13. Inhalateur doseur selon une des revendications précédentes,
**caractérisé par le fait que** l'indication d'ordre pour « Inhaler » (INHALE) ne peut être émise qu'après l'ouverture de la vanne d'alimentation en air frais 35 et/ou la fermeture de la vanne d'expiration entraînée 36.

14. Inhalateur doseur selon une des revendications précédentes,
**caractérisé par le fait que** l'indication d'ordre pour « Expirer » (EXHALE) ne peut être émise qu'après la fermeture de la vanne d'alimentation en air frais 35 et/ou l'ouverture de la vanne d'expiration entraînée 36.

15. Inhalateur doseur selon une des revendications précédentes,
**caractérisé par le fait que** la commande 4 peut émettre des ordres de façon à ce que, dans une première phase « préparation de l'aérosol », le vaporisateur produise la quantité d'aérosol nécessaire et l'amène dans le dôme aérosol 3 et à ce que le transmetteur d'ordre 5 émette le signe d'ordre pour « Expirer » (EXHALE), et dans une deuxième phase « Inhaler », le transmetteur d'ordre 5 émette le signe d'ordre pour « Inhaler » (EXHALE) et émette le signe d'ordre pour « attendre » (WAIT), et à ce qu'ensuite un autre cycle se déroule avec les trois mêmes phases.

16. Inhalateur doseur selon la revendication 15, **caractérisé par le fait que** la commande 4 peut émettre des ordres de façon à ce que dans la première phase « préparation de l'aérosol », l'obtention de la quantité d'aérosol souhaitée soit surveillée en permanence par le capteur de densité 71 et à ce que le vaporisateur 2 soit à nouveau activé si elle n'est pas atteinte.

17. Inhalateur doseur selon la revendication 15, **caractérisé par le fait que** dans la première phase le transmetteur d'ordre 5 a activé le signe d'ordre pour « Expirer », mais que la reconnaissance de respiration 72 sur le tube d'expiration 33 n'est pas active, la commande électronique 4 dans le transmetteur d'ordre d'utilisateur 5 renforce le signe d'ordre pour « Expirer », par exemple à travers le clignotement d'un affichage optique et/ou l'activation supplémentaire d'un autre affichage d'ordre, comme par exemple un signal acoustique.

18. Inhalateur doseur selon la revendication 15, **caractérisé par le fait que** dans la deuxième phase « Inspirer », la commande électronique 4 renforce le signe d'ordre pour « Inspirer » en cas d'absence de l'activation de la respiration 72, par exemple à travers le clignotement d'un affichage optique et/ou l'activation supplémentaire d'un autre affichage d'ordre, comme par exemple un signal acoustique et le signe d'ordre pour « Inspirer » après une certaine limite de temps d'aspiration pouvant être réglée.

19. Inhalateur doseur selon la revendication 18, **caractérisé par le fait que** la commande de 4 peut émettre des ordres de façon à ce que dans la seconde phase « Inspirer », si la reconnaissance de respiration 72 sur le tube d'alimentation en air frais 31 n'est que partiellement ou pas du tout active et si le capteur de densité 71 signale toujours la présence d'aérosol, la commande électronique 4 dans le transmetteur d'ordre 5 arrête, pour la stabilité de l'aérosol, le signe d'ordre pour « Inspirer » après le dépassement du temps maximal réglé selon la revendication 12.

20. Inhalateur doseur selon la revendication 19, **caractérisé par le fait qu'**après le dépassement du temps maximal pour la stabilité de l'aérosol, la commande 4 ne tient pas compte de la quantité de l'aérosol non inhalée lors du calcul de la quantité totale inhalée jusqu'à présent.
